# EUROPÄISCHE PATENTSCHRIFT

(11) **EP 2 609 849 B1**
(45) Veröffentlichungstag und Bekanntmachung des Hinweises auf die Patenterteilung: **15.03.2017**
(21) Anmeldenummer: 12008306.8
(22) Anmeldetag: 13.12.2012
(51) Int. Cl.: A61B 1/00, A61B 1/07, A61B 1/04, A61B 5/00, A61B 1/06

(54) **Vorrichtung und Verfahren zur endoskopischen Fluoreszenzdetektion**
Device and method for endoscopic fluorescence detection
Dispositif et procédé de détection de fluorescence endoscopique

(30) Priorität: 30.12.2011 DE 102011122602
(43) Veröffentlichungstag der Anmeldung: 03.07.2013
(73) Patentinhaber: Karl Storz GmbH & Co. KG, 78532 Tuttlingen (DE)
(72) Erfinder: Ehrhardt, André, Dr., 78573 Wurmlingen (DE); Göbel, Werner, Dr., 78532 Tuttlingen (DE); Irion, Klaus M., Dr.-Ing, 78576 Liptingen (DE)
(74) Vertreter: Fugmann, Winfried

(56) Entgegenhaltungen:
- EP-A1- 2 098 156
- WO-A2-02/07587
- WO-A2-02/50518
- US-A1- 2006 052 710
- US-A1- 2009 114 803
- US-A1- 2009 147 998
- US-A1- 2010 079 587
- US-B1- 6 293 911

## Beschreibung

Die vorliegende Erfindung betrifft eine Vorrichtung und ein Verfahren zur endoskopischen Fluoreszenzdetektion.

In vielen medizinischen Bereichen ist die Entnahme von Gewebeproben zur histologischen oder molekular-biologischen Untersuchung für eine sichere Diagnose unerlässlich. Hierfür wird eine Biopsienadel in ein menschliches oder tierisches Gewebe eingeführt, um an der Spitze der Biopsienadel beispielsweise durch eine durch die Biopsienadel einzuführende Biopsiezange Gewebeproben für die nachfolgenden Untersuchungen zu gewinnen. Für die Aussagekraft der Ergebnisse ist es entscheidend, an welchem Ort die Gewebeproben entnommen worden sind. Soll beispielsweise das Gewebe eines Tumors histopathologisch untersucht werden, so muss sichergestellt sein, dass die Gewebeprobe auch tatsächlich aus einem hierfür geeigneten, d.h. vitalen Bereich des Tumors entnommen wird und nicht aus dem nekrotischen Bereich des Tumors, der keine histopathologische Auswertung erlaubt. Auch eine Gewebeprobe aus einem direkt an den Tumor angrenzenden Gewebebereich wäre bei diesem Untersuchungsverfahren nicht zielführend.

So werden etwa bei einer stereotaktischen Biopsie Proben aus einem Hirntumor für weitere Untersuchungen entnommen. Aufgrund vorangegangener Untersuchungen, etwa mit Röntgen- oder NMR-Verfahren, ist die Lage des Tumors bekannt. Für eine genaue Platzierung der Biopsienadel wird ein Stereotaxiesystem verwendet, bei dem eine Führungsvorrichtung am Kopf des Patienten fixiert wird, so dass dünne Instrumente entlang eines vorher geplanten Wegs (Trajektorie) zielgenau zum vorherbestimmten Ort der Probenentnahme geführt werden können.

Auch bei einer stereotaktischen Biopsie kann jedoch trotz vorheriger Festlegung des Orts und der Trajektorie zur Probenentnahme nicht immer sicher ausgeschlossen werden, dass der zur Probenentnahme geeignete Bereich des Tumors verfehlt oder ein Blutgefäß beim Einführen der Biopsienadel verletzt wird, wodurch das Risiko von Blutungen mit ernsthaften Komplikationen besteht. Dieses Risiko wird dadurch erhöht, dass beispielsweise nach dem Öffnen der Schädeldecke eine Verschiebung des Gehirns auftreten kann. Es wäre daher wünschenswert, endoskopisch sowohl vitales Tumorgewebe als auch auf dem Weg zum Ort der Probenentnahme zu durchdringendes Gewebe, insbesondere Blutgefäße, identifizieren zu können.

Aus US 2006/0052710 A1 ist es bekannt, die Fluoreszenz des Gewebes in zwei unterschiedlichen infraroten Spektralbereichen zu detektieren. Hierfür werden zwei unterschiedliche Photosensibilisatoren verabreicht und Anregungslicht in zwei unterschiedlichen Spektralbereichen eingestrahlt. Die hierdurch angeregte Fluoreszenz in zwei unterschiedlichen infraroten Spektralbereichen ermöglicht nicht nur die Erkennung von entartetem Gewebe, sondern bei entsprechender Wahl der Photosensibilisatoren auch die Erkennung gesunden Gewebes. Hierdurch wird es ermöglicht, festzustellen, ob an einem Ort, an dem die für das Tumorgewebe charakteristische Fluoreszenzstrahlung nicht beobachtet wird, gesundes Gewebe vorliegt, oder ob lediglich die Photosensibilisatoren an dem entsprechenden Ort noch nicht in ausreichendem Maße in dem Gewebe angereichert worden sind. Hierdurch soll eine sicherere Detektion von entartetem Gewebe ermöglicht werden.

Gemäß WO 2004/106896 A2 werden zwei Paare von Fluoreszenzanregung und -emission genutzt, um zwei Fluoreszenzbilder zu erzeugen. Als erstes Anregungs-Emissions-Paar wird Anregungsstrahlung im blauen Spektralbereich verwendet, um Fluoreszenz im grünen/roten Wellenlängenbereich zu erzeugen. Das zweite Anregungsemissionspaar umfasst Anregungsstrahlung im roten/nahen Infrarotbereich und Fluoreszenzstrahlung in einem längerwelligen roten/nahen Infrarotbereich. Hierdurch soll eine verbesserte Erkennung von entartetem Gewebe ermöglicht werden.

In dem Artikel von Muldoon et al. (Optics Express 2007, Vol. 15, No. 25, Seiten 16413-16423) wird ein Mikroendoskop beschrieben, mit dem *in vivo* Acriflavinhydrochloridinduzierte Fluoreszenz beobachtet werden konnte. In einem Kollagenmodell wurden *in vitro* bei Beleuchtung durch dieselbe LED-Lichtquelle durch drei unterschiedliche Fluorophore erzeugte Fluoreszenzen sequentiell durch den Einsatz von drei separaten Emissionsfiltern nachgewiesen.

In US 6,293,911 B1 ist ein Fluoreszenzendoskopsystem zur gleichzeitigen normalen Beobachtung und zur Fluoreszenzbeobachtung im infraroten Spektrum offenbart. Dabei werden das Licht für die normale Beobachtung, das Wellenlängen im sichtbaren Spektrum aufweist, und Anregungslicht mit Wellenlängen im infraroten Spektrum, das einen Fluoreszenzstoff anregt, gleichzeitig durch ein Endoskop auf lebendes Gewebe eingestrahlt. Das Beleuchtungslicht wird über eine Lichtleitfaser, die im Einführungsteil des Endoskops verläuft, zum distalen Ende des Einführungsteils und das am distalen Ende des Einführungsteils aufgenommene Bild über einen Bildleiter zu einem Kamerakopf weitergeleitet. Ein dichroitischer Spiegel reflektiert infrarotes Licht zu einem Kantenfilter, nach dem ein Bildverstärker sowie ein CCD angeordnet sind, das das das vom Bildverstärker verstärkte infrarote Licht empfängt. Das Kantenfilter ist derart ausgebildet, dass das Fluoreszenzlicht von Antikörpern, die mit Indocyaningrün (ICG) markiert sind, zum Bildverstärker durchgelassen wird. Ein dichroitisches Prisma teilt das vom dichroitischen Spiegel durchgelassene sichtbare Licht in die Spektralbereiche rot, grün und blau auf, die zur Erzeugung eines visuellen Bilds von drei CCDs empfangen werden.

In US 2009/147998 A1 sowie in US 2009/114803 A1 ist ein Bildverarbeitungssystem beschrieben, das eingerichtet ist, um ein Bild eines Objekts innerhalb eines Körpers zu korrigieren. Durch den Endabschnitt des Endoskops wird infrarotes Licht und Beleuchtungslicht abgestrahlt, wobei das infrarote Licht zur Anregung eines Lumineszenzstoffs dient, und es werden von einer Bildaufnahmesektion Bilder im Lumineszenzlicht und im reflektierten Licht aufgenommen. Gemäß WO 02/50518 A2 wird Fluoreszenz in einem Fluoreszenzwellenlängenbereich beobachtet, der im grünen Spektralbereich liegen kann; darüber hinaus dienen vier Bildkanäle zur Detektion von reflektiertem Licht.

In EP 2 098 156 A1 ist ein Endoskopsystem offenbart, das einen Einführungsabschnitt zur Einführung in eine Körperhöhle eines Organismus, eine Bilderzeugungseinheit, eine Lichtquelleneinheit zum Erzeugen von Anregungslicht sowie Beleuchtungslicht für eine normale Beobachtung, eine Flüssigkeitszuführungseinheit, eine Steuerungseinheit und eine Anzeigeeinheit umfasst. In einem normalen Beobachtungsmodus wird ein Bild im reflektierten Licht dargestellt. In zwei Fluoreszenzbeobachtungsmoden werden die reflektierte Strahlung sowie die gesamte Fluoreszenzstrahlung zweier Fluoreszenzteststoffe bei sequentieller Einstrahlung von Beleuchtungslicht und Anregungsstrahlung bzw. Beleuchtungslicht, Anregungsstrahlung in einem ersten Anregungswellenlängenbereich und Anregungsstrahlung in einem zweiten Anregungswellenlängenbereich aufgenommen. Anstelle von Beleuchtungslicht kann auch Anregungsstrahlung zur Anregung eines dritten Fluoreszenzteststoffs oder zur Anregung von Autofluoreszenz auf das Zielgebiet abgestrahlt werden.

Gemäß WO 02/076587 A2 erfolgt durch ein Endoskop die Anregung und Beobachtung von zwei unterschiedlichen Fluoreszenzmodi, wovon einer die Autofluoreszenz ist. Ein zweites Fluoreszenzbild oder ein Bild im reflektierten Licht werden als Referenzsignal zur Normierung genutzt. Anstelle der Autofluoreszenz kann auch induzierte Fluoreszenz beobachtet werden. In US 2010/079587 A1 ist eine Anordnung von drei CCDs im distalen Endbereich eines Endoskops offenbart, um blaue Autofluoreszenz, NIR-Fluoreszenz und ein visuelles Bild aufzunehmen.

Die bekannten Verfahren und Vorrichtungen zur Fluoreszenzdetektion sind nicht ausreichend, um sowohl eine sichere Detektion des für die Probenentnahme vorgesehenen vitalen Tumorgewebes im Vergleich zu Normalgewebe und nekrotischem Tumorgewebe als auch eine sichere Erkennung von Blutgefäßen beim Einführen der Biopsienadel zu ermöglichen.

Es ist Aufgabe der vorliegenden Erfindung, eine Vorrichtung sowie ein Verfahren zur endoskopischen Fluoreszenzdetektion anzugeben, wodurch eine verbesserte Unterstützung der Einführung einer Biopsienadel zur endoskopischen Probenentnahme ermöglicht wird, um insbesondere sowohl vitales Tumorgewebe als auch Normalgewebe und Blutgefäße sicherer erkennbar zu machen.

Diese Aufgabe wird durch eine Vorrichtung sowie durch ein Verfahren gemäß den unabhängigen Ansprüchen gelöst.

Vorteilhafte Weiterbildungen der Erfindung ergeben sich aus den Unteransprüchen.

Eine erfindungsgemäße Vorrichtung zur endoskopischen Fluoreszenzdetektion umfasst eine Lichterzeugungseinrichtung zur Erzeugung einer Fluoreszenzanregungsstrahlung. Die Fluoreszenzanregungsstrahlung ist insbesondere Strahlung im sichtbaren Spektralbereich und/oder im ultravioletten und/oder im nahen Infrarotbereich; die beiden letzteren Spektralbereiche werden hier ebenfalls als "Licht" bezeichnet. Die Fluoreszenzanregungsstrah-lung kann einen oder mehrere, insbesondere mehrere voneinander getrennte, Wellenlängenbereiche innerhalb der genannten Spektralbereiche umfassen. Zur Erzeugung der Fluoreszenzanregungsstrahlung umfasst die Lichterzeugungseinrichtung eine oder mehrere Lichtquellen, die beispielsweise lichtemittierende Dioden (LEDs), Laserdioden oder Gasentladungslampen sein können.

Die Vorrichtung zur endoskopischen Fluoreszenzdetektion umfasst weiterhin einen Lichtleiter, der endoskopisch einsetzbar ist, d.h. zur Einführung in einen körperinneren Hohlraum oder auch beispielsweise in einen Arbeitskanal eines Endoskops ausgebildet ist. Der Lichtleiter ist insbesondere langerstreckt und vorzugsweise flexibel ausgebildet. Der Lichtleiter ist zur Weiterleitung der von der Lichterzeugungseinrichtung erzeugten Fluoreszenzanregungsstrahlung von einem proximalen, d.h. benutzernahen, Ende des Lichtleiters zu einem zu untersuchenden menschlichen oder tierischen Gewebe ausgebildet. Hierfür kann der Lichtleiter an seinem distalen, d.h. benutzerfernen, Ende eine Lichtaustrittsfläche aufweisen, durch die die Fluoreszenzanregungsstrahlung in Richtung auf das zu untersuchende Gewebe austritt. Der Lichtleiter ist ferner zur Aufnahme und Weiterleitung einer von dem Gewebe emittierten Fluoreszenzstrahlung zum proximalen Ende des Lichtleiters ausgebildet. Hierfür kann die Lichtaustrittsfläche auch als Lichteintrittsfläche für den Eintritt der Fluoreszenzstrahlung in das distale Ende des Lichtleiters ausgebildet sein. Der Lichtleiter kann ggf. zur Weiterleitung weiterer Strahlungsarten, beispielsweise einer weiteren Beleuchtungsstrahlung und/oder des vom Gewebe reflektierten bzw. gestreuten Lichts geeignet sein.

Ferner umfasst die erfindungsgemäße Vorrichtung eine zur Detektion der Fluoreszenzstrahlung ausgebildete Detektoreinrichtung und eine am proximalen Ende des Lichtleiters angeordnete Strahlteileroptik zur Einkopplung der Fluoreszenzstrahlung in das proximale Ende des Lichtleiters und zur Weiterleitung der Fluoreszenzstrahlung vom proximalen Ende des Lichtleiters zur Detektoreinrichtung. Die Strahlteileroptik kann hierfür eine oder mehrere strahlteilende Flächen sowie ggf. weitere optische Elemente umfassen.

Erfindungsgemäß ist die Lichterzeugungseinrichtung zur Erzeugung einer solchen Fluoreszenzanregungsstrahlung ausgebildet, die zur Anregung des zu untersuchenden Gewebes zur Emission von Fluoreszenzstrahlung in mindestens drei unterschiedlichen Fluoreszenzwellenlängenbereichen geeignet ist. Hierfür können zuvor ein oder mehrere Photosensibilisatoren verabreicht worden sein, so dass das Gewebe entsprechende Farbstoffe bzw. Fluoreszenzstoffe (Fluorophore) enthält bzw. anreichert, die die Fluoreszenzstrahlung emittieren. Die drei unterschiedlichen Fluoreszenzwellenlängenbereiche entsprechen insbesondere unterschiedlichen Fluoreszenzmodi, die etwa durch unterschiedliche Photosensibilisatoren vermittelt werden können (induzierte Fluoreszenz) oder auch eine Eigenschaft des menschlichen oder tierischen Gewebes selbst darstellen (Autofluoreszenz). Die Fluoreszenzanregungsstrahlung kann auf das Absorptionsmaximum eines oder mehrerer Fluorophore abgestimmt sein. Die Fluoreszenzstrahlung enthält somit mindestens drei Anteile in jeweils unterschiedlichen Wellenlängenbereichen. Erfindungsgemäß ist die Detektoreinrichtung weiterhin zur Detektion jedes einzelnen der drei Anteile der Fluoreszenzstrahlung in den unterschiedlichen Fluoreszenzwellenlängenbereichen und damit zur Unterscheidung der mindestens drei unterschiedlichen Fluoreszenzwellenlängenbereiche ausgebildet.

Dadurch, dass die erfindungsgemäße Vorrichtung zur endoskopischen Fluoreszenzdetektion einen endoskopisch einführbaren Lichtleiter aufweist, der zur Weiterleitung der Fluoreszenzanregungsstrahlung zum zu untersuchenden menschlichen oder tierischen Gewebe sowie zur Weiterleitung der von dem Gewebe emittierten Fluoreszenzstrahlung zur Detektion der Fluoreszenzstrahlung vorgesehen ist, wird die Detektion der Fluoreszenzstrahlung des zu untersuchenden Gewebes bei einem endoskopischen Eingriff, insbesondere bei einer endoskopischen Entnahme einer Gewebeprobe, ermöglicht. Dadurch, dass die Lichterzeugungseinrichtung zur Erzeugung einer Fluoreszenzanregungsstrahlung ausgebildet ist, die Fluoreszenzen in mindestens drei unterschiedlichen Fluoreszenzwellenlängenbereichen anregt, und die drei unterschiedlichen Fluoreszenzwellenlängenbereiche von der Detektoreinrichtung detektiert und unterschieden werden können, wird eine sicherere Unterscheidung von mindestens drei unterschiedlichen Gewebearten bzw. Arten von Zellen ermöglicht. Hierdurch können somit bei einer endoskopischen Biopsie unterschiedliche Gewebearten, Zellarten und/oder Organe in verbesserter Weise detektiert werden. Hierdurch wird es insbesondere ermöglicht, beim Einführen einer Biopsienadel Blutgefäße sicherer zu erkennen und zu vermeiden sowie durch Unterscheidung von gesundem sowie nekrotischem und vitalem Tumorgewebe sicherer festzustellen, ob der für die Probenentnahme geeignete Ort erreicht ist.

Gemäß einer bevorzugten Ausführungsform der Erfindung ist die Lichterzeugungseinrichtung zur Erzeugung einer Fluoreszenzanregungsstrahlung ausgebildet, die mindestens einen ersten und einen zweiten Anregungswellenlängenbereich umfasst. Die Fluoreszenzanregungsstrahlung im ersten Anregungswellenlängenbereich führt zur Anregung eines ersten Fluoreszenzmodus des Gewebes, und die Fluoreszenzanregungsstrahlung im zweiten Anregungswellenlängenbereich führt zur Anregung eines zweiten und eines dritten Fluoreszenzmodus des Gewebes. Die Anregung des ersten, zweiten und dritten Fluoreszenzmodus führt jeweils zur Emission von Fluoreszenzstrahlung in einem ersten, zweiten bzw. dritten Fluoreszenzwellenlängenbereich. Insbesondere sind der erste und der zweite Anregungswellenlängenbereich voneinander getrennt, und der erste Fluoreszenzwellenlängenbereich ist vom zweiten und/oder dritten Fluoreszenzwellenlängenbereich getrennt. So können beispielsweise der zweite und der dritte Fluoreszenzwellenlängenbereich zwischen dem ersten und dem zweiten Anregungswellenlängenbereich liegen und vom ersten Fluoreszenzwellenlängenbereich durch den ersten Anregungswellenlängenbereich getrennt sein. Dadurch, dass durch Fluoreszenzanregungsstrahlung in zwei Anregungswellenlängenbereichen Fluoreszenzstrahlung in drei Fluoreszenzwellenlängenbereichen erzeugt wird, wird auf einfache Weise eine ausreichende Unterscheidung unterschiedlicher Zell- bzw. Gewebearten ermöglicht, um eine sicherere Einführung einer Biopsienadel und eine sicherere Erkennung des vorgesehenen bzw. vorher berechneten Orts einer Biopsie zu ermöglichen.

In bevorzugter Weise sind der erste und der zweite Anregungswellenlängenbereich derart gewählt, dass der erste und der zweite Fluoreszenzmodus durch einen oder mehrere Photosensibilisatoren induzierte Fluoreszenzmodi sind, und der dritte Fluoreszenzmodus die Autofluoreszenz des Gewebes darstellt. Hierdurch wird durch die Gabe von lediglich zwei Photosensibilisatoren eine Detektion von drei Fluoreszenzmodi und damit eine Unterscheidung einer ausreichenden Anzahl von Zell- bzw. Gewebearten zur Unterstützung der Einführung einer Biopsienadel ermöglicht.

Insbesondere ist der erste Fluoreszenzmodus eine durch Indocyaningrün (ICG) erzeugte Fluoreszenz. Zur Anregung der ICG-Fluoreszenz ist insbesondere Fluoreszenzanregungsstrahlung im nahen Infrarotbereich mit einem Maximum bei 785 nm geeignet, d.h. im Bereich des Absorptionsmaximums des ICG. Die ICG-Fluoreszenz kann in einem Fluoreszenzwellenlängenbereich von ca. 800 bis 900 nm detektiert werden. Weiterhin ist der zweite Fluoreszenzmodus insbesondere die durch Protoporphyrin IX (PpIX) erzeugte Fluoreszenz, wofür eine Fluoreszenzanregungsstrahlung in einem Anregungswellenlängenbereich bei 405 nm, d.h. im Bereich der maximalen Absorption des PpIX, geeignet ist. Die PpIX-Fluoreszenz kann in einem Fluoreszenzwellenlängenbereich von ca. 620 bis 700 nm detektiert werden. Die für die Erzeugung der PpIX-Fluoreszenz eingestrahlte Fluoreszenzanregungsstrahlung mit einem Maximum bei 405 nm ist gleichzeitig zur Anregung der Autofluoreszenz von menschlichem oder tierischem Gewebe geeignet, die in einem dritten Fluoreszenzwellenlängenbereich von ca. 410 bis 600 nm detektiert werden kann. Die ICG- und die PpIX-Fluoreszenz können durch vorherige Gabe von ICG bzw. 5-Aminolävulinsäure (5-ALS), woraus durch Stoffwechselvorgänge das fluoreszenzfähige PpIX entsteht, induziert werden. Die genannten Photosensibilisatoren sind einfach zu verabreichen und gut verträglich, und die dadurch induzierten Fluoreszenzen ermöglichen im Zusammenwirken mit der Autofluoreszenz eine sichere Unterscheidung unterschiedlicher Zell- bzw. Gewebearten zur Unterstützung der Einführung einer Biopsienadel.

Alternativ kann als Photosensibilisator beispielsweise Fluoreszein verwendet werden. Fluoreszein kann vorzugsweise durch Fluoreszenzanregungsstrahlung mit einem Maximum bei 488 nm zur Fluoreszenz angeregt und die emittierte Fluoreszenz im grünen Spektralbereich von 500 bis 600 nm detektiert werden. Fluoreszein erlaubt insbesondere eine Detektion von Blutgefäßen.

In vorteilhafter Weise kann die Fluoreszenzanregungsstrahlung einen weiteren Anregungswellenlängenbereich umfassen, der zur Anregung von Fluoreszenzstrahlung in einem weiteren Fluoreszenzwellenlängenbereich geeignet ist, wobei diese weitere Fluoreszenz durch einen weiteren Photosensibilisator induziert wird. Hierdurch kann eine weiter verbesserte Unterscheidung unterschiedlicher Zell- bzw. Gewebearten ermöglicht werden. So kann beispielsweise zusätzlich zu ICG und 5-ALS Fluoreszein verabreicht werden, wodurch eine weiter verbesserte Detektion von Blutgefäßen ermöglicht wird.

Gemäß einer bevorzugten Ausführungsform der Erfindung umfasst die Lichterzeugungseinrichtung eine erste und eine zweite Lichtquelle, wobei die erste Lichtquelle Fluoreszenzanregungsstrahlung im ersten Anregungswellenlängenbereich und die zweite Lichtquelle Fluoreszenzanregungsstrahlung im zweiten Anregungswellenlängenbereich erzeugt. Hierdurch kann auf einfache Weise eine Fluoreszenzanregungsstrahlung in unterschiedlichen Anregungswellenlängenbereichen zur Anregung unterschiedlicher Fluoreszenzmodi erzeugt werden. Die Lichterzeugungseinrichtung kann weitere Lichtquellen umfassen, etwa zur Erzeugung einer Fluoreszenzanregungsstrahlung zur Anregung einer weiteren Fluoreszenz.

Vorzugsweise sind die erste und die zweite Lichtquelle als Laserdioden ausgebildet, die jeweils Licht in dem betreffenden Anregungswellenlängenbereich erzeugen bzw. auf die für die Anregung der entsprechenden Fluoreszenzmodi geeigneten Wellenlängenbereiche abgestimmt sind. Die Verwendung von Laserdioden ermöglicht die Erzeugung von Fluoreszenzanregungsstrahlung mit einer ausreichenden Intensität, wodurch außerdem eine besonders effektive Einkopplung der Fluoreszenzanregungsstrahlung in den Lichtleiter ermöglicht wird. Um den Wellenlängenbereich der Laserdioden auf ein möglichst schmales Band einzuschränken und evtl. auftretende Seitenbanden der Laserdioden zu unterdrücken, kann es vorteilhaft sein, ein zusätzliches Anregungsfilter in den Strahlengang nach der Laserdiode zu integrieren. Ein solches Anregungsfilter kann beispielsweise als schmalbandiges Bandpassfilter ausgeführt sein.

Weiterhin ist es vorteilhaft, dass die erste und die zweite Lichtquelle unabhängig voneinander steuerbar sind. Hierdurch wird eine unabhängige Anregung und Detektion der unterschiedlichen Fluoreszenzmodi zur Detektion unterschiedlicher Zell- bzw. Gewebearten ermöglicht, wobei die Erzeugung und Einstrahlung der Fluoreszenzanregungsstrahlung für einen aktuell nicht beobachteten Fluoreszenzmodus unterbleiben kann.

Gemäß einer besonders bevorzugten Ausführungsform der Erfindung umfasst die Strahlteileroptik mindestens einen wellenlängenselektiven Strahlteiler. Der wellenlängenselektive Strahlteiler kann insbesondere dazu ausgebildet und angeordnet sein, die Fluoreszenzanregungsstrahlung zu reflektieren und die Fluoreszenzstrahlung durchzulassen oder umgekehrt. Hierfür kann der Strahlteiler als wellenlängenselektiver bzw. dichroitischer Strahlteiler derart ausgebildet sein, dass Licht in einer Mehrzahl von unterschiedlichen Anregungswellenlängenbereichen reflektiert und in den mindestens drei unterschiedlichen Fluoreszenzwellenlängenbereichen durchgelassen wird bzw. umgekehrt. Insbesondere kann der wellenlängenselektive Strahlteiler für eine entsprechende wellenlängenselektive Reflexion unter einem Winkel von 45° zu einer Spiegelfläche des Strahlteilers ausgebildet sein. Hierdurch wird auf besonders einfache Weise eine effiziente Einkopplung der Fluoreszenzanregungsstrahlung in den Lichtleiter und eine Weiterleitung der Fluoreszenzstrahlung in den mindestens drei unterschiedlichen Fluoreszenzwellenlängenbereichen mit geringen Lichtverlusten zur Detektoreinrichtung ermöglicht.

Weiterhin ist es bevorzugt, dass die Strahlteileroptik einen oder mehrere Blockfilter aufweist, die bei einer ggf. unvollständigen spektralen Trennung durch den wellenlängenselektiven Strahlteiler einen durch den Strahlteiler hindurch gehenden Restanteil der am Gewebe oder auch etwa innerhalb des Lichtleiters reflektierten oder rückgestreuten Fluoreszenzanregungsstrahlung auf dem Weg zur Detektoreinrichtung abblocken. Der bzw. die Blockfilter sind zu diesem Zweck insbesondere in den Anregungswellenlängenbereichen undurchlässig. Ein solcher Blockfilter kann beispielsweise als Kerbfilter ausgebildet sein, der Licht in einem oder mehreren schmale Wellenlängenbereichen absorbiert und ansonsten eine hohe Transmission aufweist. Dies ist vor allem in dem Fall vorteilhaft, dass Laserdioden als Lichtquellen verwendet werden, da durch einen Kerbfilter der ggf. durch den Strahlteiler durchgehende Teil der von einer Laserdiode erzeugten schmalbandigen Fluoreszenzanregungsstrahlung effektiv abgeblockt werden kann, ohne dass ein wesentlicher Verlust an Fluoreszenzstrahlung auftritt. Ein Blockfilter kann auch beispielsweise als Langpassfilter ausgebildet sein.

Gemäß einer bevorzugten Ausführungsform der Erfindung ist zumindest ein distaler Endabschnitt des Lichtleiters als endoskopisch einführbare Sonde ausgebildet oder in einer endoskopisch einführbaren Sonde aufgenommen. Hierfür kann insbesondere der distale Endabschnitt des Lichtleiters in einem langerstreckten, endoskopisch einführbaren Schaft angeordnet bzw. gefasst sein. Der Lichtleiter kann beispielsweise zweiteilig ausgebildet sein, wobei ein distaler Teil des Lichtleiters die Sonde bildet bzw. in der Sonde aufgenommen ist und wobei am proximalen Ende eines proximalen Teils die Strahlteileroptik angeordnet ist. Das proximale Ende des distalen Teils des Lichtleiters ist dabei mit dem distalen Ende des proximalen Teils des Lichtleiters über eine Kupplung zur bidirektionalen Lichtübertragung verbindbar. Die Sonde kann auch beispielsweise als Schaft eines miniaturisierten Endoskops ausgebildet sein, das weiterhin die Strahlteileroptik sowie Anschlüsse zum Anschließen der Detektoreinrichtung und der Lichterzeugungseinrichtung aufweist. Die Sonde bzw. der Endoskopschaft kann hierbei besonders dünn ausgeführt sein, weil der Lichtleiter sowohl zur Weiterleitung der Fluoreszenzanregungsstrahlung von proximal nach distal wie auch zur Weiterleitung der Fluoreszenzstrahlung von distal nach proximal dient und daher hierfür keine zwei getrennten Lichtleiter erforderlich sind. Dadurch, dass zumindest der distale Endabschnitt des Lichtleiters als endoskopisch einführbare Sonde ausgebildet bzw. in einer solchen aufgenommen ist, wird ein endoskopisches Einführen des Lichtleiters erleichtert.

In vorteilhafter Weise ist die Sonde zum Einsetzen in eine Biopsienadel ausgebildet und weist hierfür einen entsprechenden Durchmesser und eine entsprechende Länge auf, wobei es besonders vorteilhaft ist, dass die Sonde anstelle eines Dorns in die Biopsienadel eingeführt werden kann. Hierfür beträgt der Durchmesser der Sonde vorzugsweise weniger als ca. 1,5 mm. Hierdurch wird es ermöglicht, die Biopsienadel in gewohnter Weise zum Ort der Probenentnahme vorzuschieben, wobei das distale Ende der Biopsienadel statt durch den üblicherweise vorgesehenen Dorn (Mandrin) durch das distale Ende der Sonde verschlossen ist. Das distale Ende der Sonne kann insbesondere derart ausgebildet sein, dass sowohl die Abgabe der Fluoreszenzanregungsstrahlung in Richtung auf das Gewebe und die Aufnahme der Fluoreszenzstrahlung vom Gewebe ermöglicht wird, als auch das Gewebe beim Vorschieben der Biopsienadel möglichst wenig geschädigt wird. Vorzugsweise ist die Sonde auch hinsichtlich ihrer Steifigkeit und der Form des distalen Endes der Sonde dem üblicherweise verwendeten Dorn ähnlich. Besonders bevorzugt ist es, dass die Sonde zum Einsetzen in eine Standard-Biopsienadel ausgebildet ist. Hierdurch wird für den Arzt das Einführen der Biopsienadel zur endoskopischen Probenentnahme in gewohnter Weise ermöglicht. Ist der Ort der Probenentnahme erreicht, so wird die Sonde aus der Biopsienadel herausgezogen und zur Probenentnahme eine Biopsiezange durch die Biopsienadel bis zum distalen Ende eingeführt.

Weiterhin ist es bevorzugt, dass der Lichtleiter als Bildleiter ausgebildet ist und die Detektoreinrichtung mindestens einen Bildsensor zur Aufnahme eines Fluoreszenzbildes umfasst. Der Bildleiter kann insbesondere als geordnetes (kohärentes) Faserbündel ausgebildet sein, wodurch auch bei dem geringen, innerhalb einer Biopsienadel zur Verfügung stehenden Durchmesser die Übermittlung eines Bilds mit einer ausreichenden Anzahl von Bildpunkten ermöglicht wird. Der Bildsensor kann beispielsweise als CCD-Bildsensor ausgebildet sein. Die Strahlteileroptik und der Bildsensor sind derart angeordnet, dass eine proximale Endfläche des Bildleiters auf die lichtempfindliche Fläche des Bildsensors abgebildet wird; hierfür kann eine Abbildungsoptik vorgesehen sein. Hierdurch wird eine räumlich aufgelöste Detektion der Fluoreszenz und damit eine verbesserte Erkennung der unterschiedlichen Arten des Gewebes ermöglicht. Insbesondere kann die Fluoreszenzstrahlung von unterschiedlichen Bereichen des an einer distalen Endfläche des Bildleiters oder einer distalen Lichtaus- und -eintrittsfläche der Sonde anliegenden Gewebes durch unterschiedliche Lichtleitfasern zum Bildsensor weitergeleitet werden, so dass ein unterschiedliches Fluoreszenzverhalten der unterschiedlichen Bereiche erkennbar ist.

Zusätzlich oder alternativ kann auch eine spektrale Analyse des remittierten Fluoreszenzlichtes erfolgen. So zeigt beispielsweise PpIX einen charakteristischen Fluoreszenz-Emissionspeak bei ca. 635 nm, ICG bei ca. 835 nm. Eine zusätzliche spektrale Analyse ermöglicht die Gewinnung zusätzlicher Informationen über die Fluoreszenzeigenschaften des untersuchten Gewebes und kann damit die Grundlage für eine Diagnose weiter verbessern. Dabei kann etwa eine Verhältnisbildung der Intensitäten der Fluoreszenzstrahlung bei unterschiedlichen Wellenlängen bzw. in verschiedenen Spektralbereichen quantitative Aussagen über die Konzentration der Fluorophore ermöglichen. Die Detektoreinrichtung kann hierfür in bevorzugter Weise ein Spektrometer, beispielsweise ein Faserspektrometer, umfassen. Die spektrale Analyse des Fluoreszenzlichtes kann insbesondere simultan zu der Erfassung eines Fluoreszenzbilds durch einen Bildsensor erfolgen. Dabei wird vorteilhafterweise ein Teil des Fluoreszenzlichtes aus dem Strahlengang des Bildsensors ausgekoppelt und auf einen zweiten, beispielsweise als Spektrometer ausgeführten Detektor, umgelenkt.

In besonders bevorzugter Weise ist am distalen Ende des Lichtleiters ein Objektiv zum Erzeugen eines Bilds des Gewebes auf einer distalen Endfläche des Bildleiters angeordnet. Das Objektiv kann insbesondere als stabförmige Gradientenlinse (GRIN-Linse) ausgebildet sein. Das auf der distalen Endfläche des Bildleiters erzeugte Bild wird durch den Bildleiter zu einer proximalen Endfläche weitergeleitet, von wo es durch die Strahlteileroptik und ggf. die Abbildungsoptik auf dem mindestens einen Bildsensor abgebildet wird. Hierdurch wird eine weiter erhöhte Auflösung somit eine verbesserte Erkennung unterschiedlicher Arten von Gewebe bzw. Organen ermöglicht, beispielsweise von Blutgefäßen und/oder Tumorgewebe.

Die Sonde kann insbesondere als Kontaktsonde ausgebildet sein. In diesem Fall wird ein Bild der Kontaktfläche des Gewebes mit der Sonde vom Bildleiter aufgenommen und zur Fluoreszenzdetektion weitergeleitet. Dabei kann das Gewebe an der distalen Endfläche des Bildleiters anliegen oder die Kontaktfläche des Gewebes kann durch das Objektiv auf die distale Endfläche des Bildleiters abgebildet werden. Hierdurch wird eine besonders einfache Verwendung der Sonde zur Detektion der Fluoreszenz des Gewebes ermöglicht.

Ferner ist es bevorzugt, dass die Vorrichtung Anzeigemittel umfasst zur Anzeige mindestens eines Fluoreszenzbildes. Insbesondere können mehrere Anzeigemittel, beispielsweise eine Mehrzahl an Bildschirmen zur Anzeige jeweils eines Fluoreszenzbildes in den mindestens drei Fluoreszenzwellenlängenbereichen vorgesehen sein. Es kann aber auch beispielsweise ein Bildschirm zur Anzeige der Fluoreszenzbilder in mehreren oder allen der mindestens drei Fluoreszenzwellenlängenbereichen vorgesehen sein, wobei die mehreren Fluoreszenzbilder nacheinander oder gleichzeitig dargestellt werden können. Bei einer gleichzeitigen Anzeige mehrerer Fluoreszenzbilder ist insbesondere eine Darstellung in unterschiedlichen Farben vorteilhaft. Hierdurch wird eine weiter verbesserte Erkennung der unterschiedlichen Gewebearten durch eine simultane oder nahezu simultane Visualisierung der unterschiedlichen Fluoreszenzen, insbesondere der PpIX-, der ICG- und der Autofluoreszenz des Gewebes, ermöglicht.

Gemäß einer weiteren Ausführungsform der Erfindung umfasst die Lichterzeugungseinrichtung eine Mehrzahl an Lichtquellen zur Erzeugung von Fluoreszenzanregungsstrahlung in einer Mehrzahl von Anregungswellenlängenbereichen, wobei die Lichtquellen sequentiell ansteuerbar sind und die Ansteuerung mit einer Aufnahmefrequenz eines Bildaufnehmers der Detektoreinrichtung synchronisierbar ist. Hierfür kann eine entsprechend eingerichtete Steuerungseinrichtung vorgesehen sein. Hierdurch ist eine Aufnahme der unterschiedlichen Fluoreszenzen sowie eine Darstellung als Farbbilder bei einem verringerten Energieeintrag in das Gewebe möglich; ferner kann hierdurch ein Ausbleichen der Fluorophore verhindert oder verringert werden.

Bei einem erfindungsgemäßen Verfahren zur endoskopischen Fluoreszenzdetektion wird eine Fluoreszenzanregungsstrahlung erzeugt, durch eine am proximalen Ende eines endoskopisch einsetzbaren Lichtleiters angeordnete Strahlteileroptik in ein proximales Ende des Lichtleiters eingekoppelt und über diesen zu einem distalen Ende den Lichtleiters und von dort zu einem zu untersuchenden menschlichen oder tierischen Gewebe weitergeleitet. Weiterhin wird eine von dem Gewebe emittierte Fluoreszenzstrahlung von dem distalen Ende des Lichtleiters aufgenommen, zum proximalen Ende des Lichtleiters weitergeleitet und durch die Strahlteileroptik zu einer Detektoreinrichtung weitergeleitet, wo die Fluoreszenzstrahlung detektiert wird. Durch die Fluoreszenzanregungsstrahlung wird erfindungsgemäß eine Emission von Fluoreszenzstrahlung des Gewebes in mindestens drei unterschiedlichen Fluoreszenzwellenlängenbereichen angeregt. Die Fluoreszenzstrahlung umfasst somit mindestens drei Anteile, die den mindestens drei Fluoreszenzwellenlängenbereichen entsprechen. Jeder der mindestens drei Anteile wird einzeln bzw. separat somit voneinander unterscheidbar detektiert.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 in schematischer Darstellung ein Ausführungsbeispiel einer erfindungsgemäßen Vorrichtung zur Fluoreszenzdetektion;
Fig. 2 in schematischer Darstellung ein weiteres Ausführungsbeispiel einer erfindungsgemäßen Vorrichtung zur Fluoreszenzdetektion;
Fig. 3 zwei Beispiele für Transmissionsfunktionen der Strahlteileroptik, wobei drei unterschiedliche Fluoreszenzwellenlängenbereiche eingetragen sind;
Fig. 4 die spektralen Empfindlichkeitsfunktionen der drei Farbkanäle einer Farbvideokamera, die zur Detektion der Fluoreszenzstrahlung in den drei unterschiedlichen Fluoreszenzwellenlängenbereichen gemäß einem Ausführungsbeispiel der Erfindung eingesetzt werden kann.

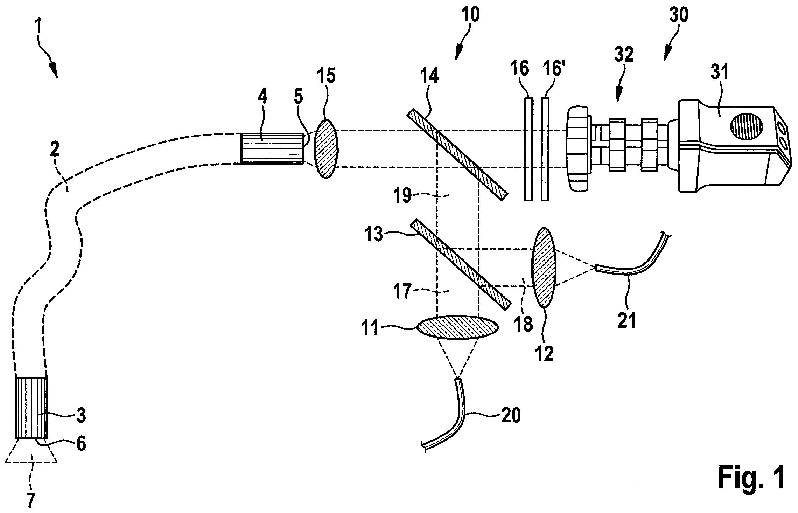

Wie in Fig. 1 schematisch gezeigt, umfasst eine erfindungsgemäße Vorrichtung gemäß dem dargestellten Ausführungsbeispiel zur endoskopischen Fluoreszenzdetektion einen Lichtleiter 1, der als flexibles geordnetes (kohärentes) Faserbündel 2 ausgebildet ist. Ein distaler Endabschnitt des Lichtleiters 1 bildet eine Sonde 3, die langerstreckt und mit einem geringen Durchmesser ausgebildet ist und zur Einführung anstelle eines Dorns in eine Biopsienadel geeignet ist (in Fig. 1 nur symbolisch angedeutet). Das distale Ende der Sonde 3 kann in eine Metallhülse mit Tiefenanschlag eingeklebt sein (nicht dargestellt). Die distale Endfläche 6 des Faserbündels 2 wird durch die miteinander verklebten und polierten Fasern gebildet. Die distale Endfläche 6 kann eine blanke Endfläche ohne weitere Abdeckung sein. Das Faserbündel 2 und insbesondere die Sonde 3 können einen Außendurchmesser von weniger als 1,5 mm, bevorzugt unterhalb von 1,0 mm, besonders bevorzugt von ca. 0,5 mm aufweisen. Ein solches Faserbündel kann beispielsweise ca. 30000 Fasern aufweisen, wobei der Abstand der einzelnen Fasermittelpunkte in den Endflächen ca. 4 µm beträgt.

Die erfindungsgemäße Vorrichtung umfasst ferner eine Strahlteileroptik 10, die in dem in Fig. 1 gezeigten Ausführungsbeispiel eine erste Kollimatoroptik 11, eine zweite Kollimatoroptik 12, einen ersten Strahlteiler 13, einen zweiten Strahlteiler 14, eine Koppeloptik 15 und einen oder mehrere Blockfilter 16, 16' umfasst. Von einer in Fig. 1 nicht näher gezeigten Lichterzeugungseinrichtung wird in dem dargestellten Ausführungsbeispiel Fluoreszenzanregungsstrahlung in einem ersten Anregungswellenlängenbereich bei 785 nm und in einem zweiten Anregungswellenlängenbereich bei 405 nm erzeugt. Hierfür kann beispielsweise jeweils eine Laserdiode vorgesehen sein, die auf die entsprechende Wellenlänge abgestimmt ist und schmalbandige Fluoreszenzanregungsstrahlung hoher Intensität bei 785 nm bzw. 405 nm erzeugt. Durch nicht dargestellte Einkopplungsoptiken wird die von den jeweiligen Laserdioden erzeugte Anregungsstrahlung in Beleuchtungslichtleiter 20, 21 eingekoppelt, aus deren Enden wie in Fig. 1 angedeutet die jeweilige Fluoreszenzanregungsstrahlung austritt, durch die erste Kollimatoroptik 11 bzw. die zweite Kollimatoroptik 12 in ein erstes bzw. zweites Anregungslichtbündel 17, 18 kollimiert wird und auf den ersten Strahlteiler 13 auftrifft. Der erste Strahlteiler 13 kann ein nicht-wellenlängenselektiver Strahlteiler sein, ist jedoch vorzugsweise als dichroitischer Spiegel ausgebildet, der bei einem jeweiligen Einfallswinkel von 45° die Anregungsstrahlung im ersten Anregungswellenlängenbereich (im Beispiel bei 785 nm) durchlässt, jedoch die Anregungsstrahlung im zweiten Anregungswellenlängenbereich (405 nm) reflektiert. Der erste Strahlteiler 13 ist derart angeordnet, dass die beiden aufgeweiteten Anregungslichtbündel 17, 18 in ein gemeinsames Anregungslichtbündel 19 vereinigt werden, das die Fluoreszenzanregungsstrahlung in den zwei Anregungswellenlängenbereichen enthält. Die Fluoreszenzanregungsstrahlung könnte aber auch etwa durch eine Xenon-Lampe erzeugt werden.

Das Anregungslichtbündel 19 trifft unter einem Einfallswinkel von 45° auf den zweiten Strahlteiler 14, der derart ausgebildet und angeordnet ist, dass das Anregungslichtbündel 19 in Richtung auf die Koppeloptik 15 reflektiert und durch diese in den proximalen Endabschnitt 4 des Faserbündels 2 eingekoppelt wird. Durch eine leichte Dekollimierung kann eine besonders gleichmäßige Ausleuchtung der proximalen Endfläche 5 und damit des Faserbündels 2 erreicht werden. Das Faserbündel 2 leitet die Fluoreszenzanregungsstrahlung über den als Sonde 3 ausgebildeten distalen Endabschnitt zu einer distalen Endfläche 6 weiter, wo die Fluoreszenzanregungsstrahlung aus dem Lichtleiter 1 austritt (in Fig. 1 symbolisch als Lichtkegel 7 dargestellt). Die Sonde 3 kann insbesondere als Kontaktsonde ausgebildet sein, so dass beim Einführen einer Biopsienadel in Gewebe, wobei die Sonde 3 als Dorn innerhalb der Biopsienadel genutzt wird, das Gewebe an der distalen Endfläche 6 anliegt. Der distale Endabschnitt 3 des Lichtleiters kann ein Objektiv umfassen, das beispielsweise als GRIN-Linse ausgebildet sein kann (in Fig. 1 nicht dargestellt).

Aufgrund der auf das Gewebe gerichteten Fluoreszenzanregungsstrahlung wird vom Gewebe Fluoreszenzstrahlung emittiert, die durch die distale Endfläche 6 in die Sonde 3 eintritt und durch den Lichtleiter 1 zu dessen proximalem Endabschnitt 4 weitergeleitet wird. Die aus der proximalen Endfläche 5 des Lichtleiters 2 austretende Fluoreszenzstrahlung wird von der Koppeloptik 15 auf den zweiten Strahlteiler 14 gerichtet. Der zweite Strahlteiler 14 ist für die Fluoreszenzstrahlung zumindest teilweise durchlässig, so dass zumindest ein Teil der Fluoreszenzstrahlung durchgelassen wird und zu einer Detektoreinrichtung 30 gelangt.

Von dem zu untersuchenden Gewebe bzw. von optischen Grenzflächen innerhalb des Lichtleiters und der Koppeloptik 15 reflektierte Fluoreszenzanregungsstrahlung wird von dem zweiten Strahlteiler 14 in Richtung des Anregungslichtbündels 19 zurückgeworfen. Da das Anregungslicht in der Regel wesentlich heller ist als das Fluoreszenzlicht, kann bei einer unvollständigen spektralen Trennschärfe des zweiten Strahlteilers 14 ein Teil der reflektierten bzw. rückgestreuten Fluoreszenzanregungsstrahlung zur Detektoreinrichtung 30 gelangen. Um zu verhindern, dass hierdurch die Detektion der Fluoreszenzstrahlung erschwert wird, sind ein oder mehrere Blockfilter 16, 16' vorgesehen, die in den Wellenlängenbereichen der Fluoreszenzanregungsstrahlung undurchlässig sind, jedoch die Fluoreszenzstrahlung möglichst ungehindert durchlassen. Bei einer schmalbandigen Fluoreszenzanregungsstrahlung, die etwa durch Laserdioden erzeugt werden kann, können die Blockfilter 16, 16' in vorteilhafter Weise als Kerbfilter ausgebildet sein, die nur einen geringen Teil des Spektrums, der der jeweiligen Anregungswellenlänge entspricht, abblocken. Insbesondere kann ein erster Blockfilter 16 zum Blocken des ersten Anregungswellenlängenbereichs (bei dem beschriebenen Ausführungsbeispiel bei 785 nm) und ein zweiter Blockfilter 16' zum Abblocken des zweiten Anregungswellenlängenbereichs (im vorliegenden Fall bei 405 nm) vorgesehen sein. Da auf der kurzwelligen Seite der zweiten Anregungswellenlänge keine Fluoreszenzstrahlung emittiert wird, kann der zweite Blockfilter 16' auch als Langpassfilter, insbesondere als Kantenfilter mit einer Kante beispielsweise zwischen ca. 410 nm und ca. 450 nm ausgebildet sein.

Wie in Fig. 1 gezeigt, umfasst die Detektoreinrichtung 30 im dargestellten Ausführungsbeispiel eine CCD-Kamera 31, die in vorteilhafter Weise als Farbvideokamera ausgebildet sein kann. Die CCD-Kamera 31 kann eine nicht dargestellte Abbildungsoptik zur Erzeugung eines Bilds der proximalen Endfläche 5 des Faserbündels 2 auf dem Bildsensor der CCD-Kamera 31 enthalten. Wie in Fig. 1 angedeutet, kann eine Kupplung 32 zur lösbaren Ankopplung der CCD-Kamera 31 an die Strahlteileroptik 10 vorgesehen sein. Die Sonde 3 kann, insbesondere gemeinsam mit der Strahlteileroptik 10, als Kontaktendoskop ausgebildet und autoklavierbar sein.

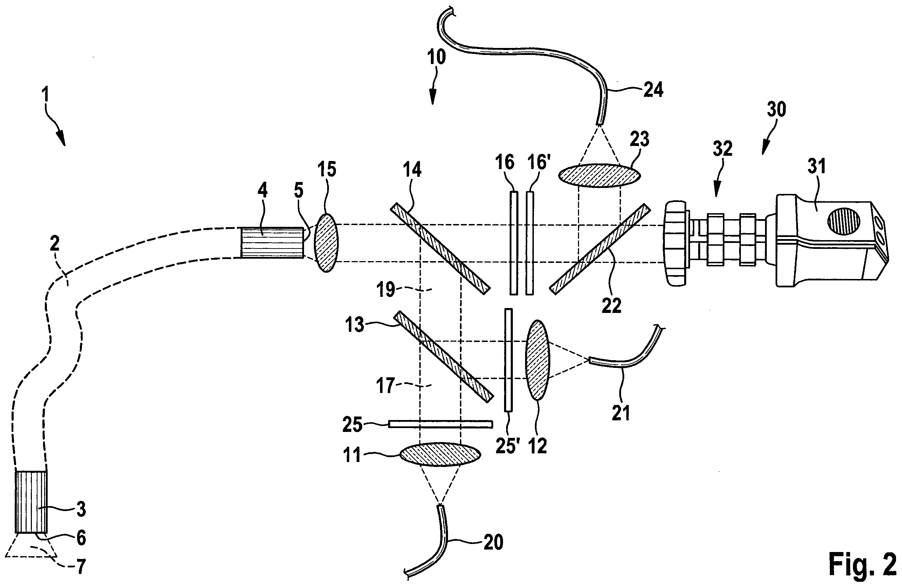

In Fig. 2 ist in schematischer Darstellung ein weiteres Ausführungsbeispiel einer erfindungsgemäßen Vorrichtung zur Fluoreszenzdetektion dargestellt, wobei zwischen den Blockfiltern 16, 16' und der CCD-Kamera 31 ein weiterer Strahlteiler 22 eingeschaltet ist, durch den ein Anteil des vom zweiten Strahlteiler 14 und den Blockfiltern 16, 16' durchgelassenen Lichts aus dem zur CCD-Kamera 31 gerichteten Strahlenbündel ausgekoppelt wird. Der ausgekoppelte Strahlungsanteil wird einer Spektralanalyse durch ein in Fig. 2 nicht dargestelltes Spektrometer zugeführt. Das Spektrometer kann insbesondere als Faserspektrometer ausgebildet sein; hierfür kann der ausgekoppelte Strahlungsanteil über eine Koppeloptik 23 in einen weiteren Lichtleiter 24, beispielsweise eine Lichtleitfaser, eingekoppelt werden. Der weitere Strahlteiler 22 kann ein nicht-wellenlängenselektiver Strahlteiler sein, der das unter 45° auftreffende Licht beispielsweise im Verhältnis 50:50 teilt. Neben der Aufnahme und visuellen Darstellung eines Fluoreszenzbilds kann somit auch eine spektrale Analyse des Fluoreszenzlichts erfolgen. Eine Spektralanalyse ermöglicht noch genauere Aussagen über die im Gewebe vorhandenen Fluorophore; so können beispielsweise durch Auswertung der Fluoreszenzintensitäten in verschiedenen Spektralbereichen quantitative Aussagen über die Fluorophorkonzentrationen ermöglicht werden.

Gemäß Fig. 2 sind in den Beleuchtungsstrahlengängen, beispielsweise nach den Kollimatoroptiken 11, 12, zusätzliche Anregungsfilter vorgesehen, die als schmalbandige Bandpassfilter 25, 25' ausgebildet sind. Hierdurch wird die eingekoppelte Fluoreszenzanregungsstrahlung auf einen besonders schmalen Wellenlängenbereich eingeschränkt, wodurch die spektrale Trennung der Fluoreszenzstrahlung von der rückgestrahlten Fluoreszenzanregungsstrahlung durch den zweiten Strahlteiler 14 und die Blockfilter 16, 16' weiter verbessert wird. Die Bandpassfilter 25, 25' können, unabhängig von dem Vorhandensein des weiteren Strahlteilers 22 und eines Spektrometers, auch in dem Ausführungsbeispiel der Fig. 1 vorgesehen sein.

Im Übrigen ist die in Fig. 2 dargestellte Vorrichtung wie die in Fig. 1 gezeigte aufgebaut, wobei der Übersichtlichkeit halber in Fig. 2 nicht alle Bezugszeichen dargestellt sind.

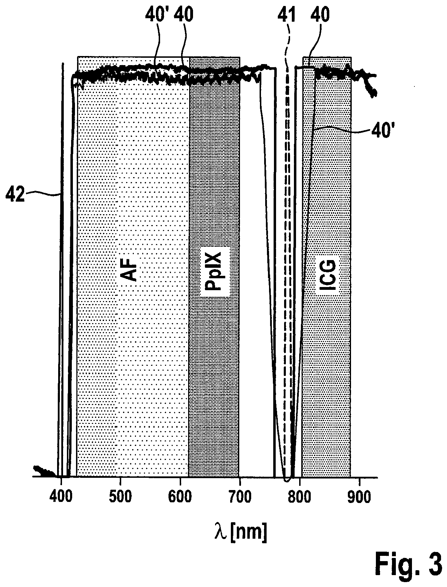

In Fig. 3 ist die spektrale Transmission der Strahlteileroptik, die durch die Durchlässigkeit des zweiten Strahlteilers und der Blockfilter bestimmt wird, beispielhaft dargestellt. Die Strahlteileroptik ist in den Wellenlängenbereichen, in denen die Fluoreszenzstrahlung von Gewebe emittiert wird, in Richtung zur Detektoreinrichtung durchlässig, jedoch in den Wellenlängenbereichen der Anregungsstrahlung undurchlässig. Die von dem Fluoreszenzfarbstoff ICG emittierte Fluoreszenzstrahlung liegt im Bereich zwischen etwa 800 und 880 nm, die Fluoreszenzstrahlung des PpIX wird zwischen etwa 600 und 700 nm, insbesondere im Bereich von ca. 635 nm, emittiert, und die Fluoreszenzstrahlung der Autofluoreszenz (AF) des Gewebes liegt etwa im Bereich zwischen 500 und 600 nm. In diesen Bereichen weist die Strahlteileroptik eine hohe Transmission auf, idealerweise nahe 100%. In den Wellenlängenbereichen der Fluoreszenzanregungsstrahlung ist die Transmission andererseits nahe 0%, um reflektierte bzw. rückgestreute Anregungsstrahlung abzublocken. Die blockierten Spektralbereiche sind möglichst schmal, um die gesamte Transmission der Fluoreszenzstrahlung möglichst wenig zu beeinträchtigen. In Fig. 3 sind zwei Beispiele für spektrale Transmissionsfunktionen 40, 40' der Strahlteileroptik dargestellt, die geringfügig unterschiedliche Transmissionen bzw. unterschiedlich breite Sperrbereiche aufweisen.

Ferner sind in Fig. 3 die spektralen Transmissionsfunktionen 41, 42 der in den Anregungsstrahlengang eingeschalteten Bandpassfilter 25, 25' angedeutet. Der Bandpassfilter 25' weist beispielsweise eine möglichst vollständige Transmission im Bereich von 400 bis 410 nm auf, während insbesondere im Bereich von 410 bis ca. 700 nm das durchgehende Licht möglichst vollständig abgeblockt wird; entsprechende Eigenschaften weist der Bandpassfilter 25 im Bereich bei 785 nm auf. In den Wellenlängenbereichen bei 405 nm bzw. 785 nm weist der zweite Strahlteiler 14 eine möglichst vollständige Reflexion zur Einkopplung der Fluoreszenzanregungsstrahlung in den Lichtleiter 1 auf.

Bei Beobachtung eines weiteren Fluoreszenzmodus kann ein weiterer Bereich geringer Transmission und hoher Reflexion vorgesehen sein (in Fig. 3 nicht dargestellt). Soll beispielsweise zusätzlich zur ICG-, PpIX- und Autofluoreszenz die Fluoreszenz von Fluoreszein beobachtet werden, so wird vorteilhafterweise eine weitere Fluoreszenzanregungsstrahlung mit einem Maximum bei 488 nm eingekoppelt. Hierfür können eine weitere Lichtquelle sowie entsprechende optische Elemente zur Einkopplung in den Lichtleiter 1 vorgesehen sein. Für Strahlung im Bereich um 488 nm ist die Transmission des zweiten Strahlteilers in diesem Fall nahe 0%, während die Reflexion nahezu 100% beträgt. Die Fluoreszenz von Fluoreszein kann im grünen Spektralbereich zwischen 500 und 600 nm beobachtet werden. In diesem Fall würde durch Fluoreszenzanregungsstrahlung in drei Anregungswellenlängenbereichen mit jeweiligen Maxima bei 405 nm, 488 nm und 785 nm Autofluoreszenz, PpIX-, Fluoreszein- und ICG-Fluoreszenz angeregt. Die Autofluoreszenz wird in diesem Fall zwischen 410 und 480 nm, die Fluoreszein-Fluoreszenz zwischen 500 und 600 nm, die PpIX-Fluoreszenz zwischen 600 und 700 nm, und die ICG-Fluoreszenz zwischen 800 und 900 nm detektiert.

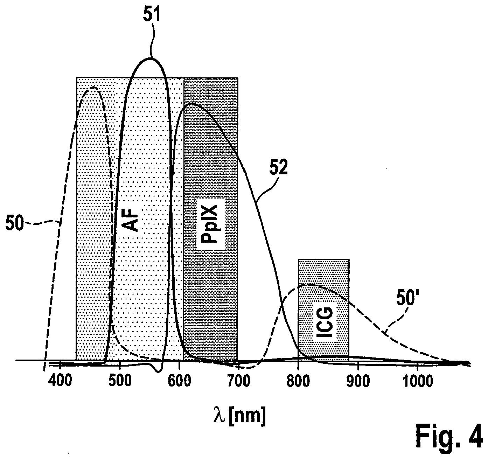

In Fig. 4 sind beispielhaft die spektralen Empfindlichkeitsfunktionen der CCD-Kamera dargestellt, die drei Farbkanäle (blau, grün und rot) aufweist. Die spektrale Empfindlichkeitsfunktion 50, 51 bzw. 52 des blauen, grünen bzw. roten Kanals ist in Fig. 4 in relativen Einheiten aufgetragen. Wie in Fig. 4 zu erkennen, weist der blaue Farbkanal (Empfindlichkeitsfunktion 50) außer dem blauen Empfindlichkeitsbereich zwischen etwa 400 nm und 500 nm einen weiteren Empfindlichkeitsbereich im infraroten Spektralbereich auf, der von ca. 750 bis ca. 1050 nm reicht. Die von dem Fluoreszenzfarbstoff ICG emittierte Fluoreszenzstrahlung kann daher durch den infraroten Empfindlichkeitsbereich des blauen Farbkanals detektiert werden. Die Fluoreszenzstrahlung des PpIX wird durch den roten Farbkanal der CCD-Kamera (Empfindlichkeitsfunktion 52) detektiert, während die Autofluoreszenzstrahlung des Gewebes (AF) vorwiegend durch den grünen Farbkanal (Empfindlichkeitsfunktion 51) detektiert wird. Durch die drei Farbkanäle der CCD-Kamera können somit die drei unterschiedlichen Fluoreszenzmodi, die zur Aussendung von Fluoreszenzstrahlung in drei unterschiedlichen Wellenlängenbereichen führen, einzeln detektiert und voneinander unterschieden werden. Die überwiegend im grünen Farbkanal detektierte Autofluoreszenz kann leicht von der ICG-Fluoreszenz im Infraroten, die ausschließlich im blauen Farbkanal detektiert wird, unterschieden werden.

Die CCD-Kamera kann einen oder mehrere Bildsensoren umfassen. In besonders vorteilhafter Weise kann etwa eine 3-Chip-Kamera verwendet werden; eine derartige Kamera wird von der Karl Storz GmbH & Co. KG unter der Bezeichnung "Tricam" angeboten. In dem Fall, dass mehr als drei Fluoreszenzmodi detektiert werden sollen, etwa zusätzlich zu ICG, PpIX und Autofluoreszenz auch die Fluoreszenz von Fluoreszein, kann eine Kamera mit vier Farbkanälen (beispielsweise rot, grün, blau und nahes Infrarot) vorteilhaft sein oder auch etwa eine 2-Chip-Kamera mit einen Farb-Bildsensor sowie einem Bildsensor für die Detektion des nahen Infrarot. Da die Autofluoreszenz in der Regel im gesamten blaugrünen Spektralbereich detektiert wird, kann diese von der ausschließlich im Grünkanal detektierten Fluoreszenz des Fluoreszein unterschieden werden.

Die Vorrichtung zur Fluoreszenzdetektion kann weiterhin eine nicht in Fig. 1 und 2 dargestellte Anzeigeeinrichtung umfassen, etwa einen Farbbildschirm, der in der üblichen Weise an die drei Farbkanäle der Videokamera angeschlossen ist. Dieser erlaubt eine gleichzeitige Darstellung von Fluoreszenzbildern, die den drei unterschiedlichen Fluoreszenzen entsprechen, in den Farben der jeweiligen Farbkanäle. Dabei erscheint die ICG-Fluoreszenz, die insbesondere zur Erkennung der Blutgefäße genutzt werden kann, blau, die PpIX-Fluoreszenz, die insbesondere vitales Tumorgewebe erkennbar macht, rot, und die Autofluoreszenz, die insbesondere gesundes Gewebe anzeigt und beispielsweise von nekrotischem Tumorgewebe zu unterscheiden gestattet, grün. Es ist aber auch möglich, die einzelnen Fluoreszenzbilder sequentiell anzuzeigen; die Lichtquellen können dabei entsprechend angesteuert werden, so dass stets nur Anregungsstrahlung in einem solchen Anregungswellenlängenbereich in das Gewebe eingestrahlt wird, der zur Anregung der jeweils aktuell beobachteten Fluoreszenz geeignet ist. Insbesondere können die Lichtquellen synchron mit den Farbmodi der Farbvideokamera angesteuert werden. Die Fluoreszenzbilder können auch zusammen mit zuvor gewonnenen 3D-Daten angezeigt werden.

Bei der Durchführung einer Biopsie mit Unterstützung durch die erfindungsgemäße Vorrichtung kann folgendermaßen vorgegangen werden:
Vor Durchführung der Biopsie werden 3D-Daten aufgenommen, insbesondere durch CT- oder NMR-Verfahren, die eine möglichst genaue Lokalisation des Tumors und ggf. weiterer Strukturen des Gewebes ermöglichen. Aufgrund dieser Daten kann eine Trajektorie geplant werden, über die der Tumor zur Entnahme der Biopsie mit einer Biopsienadel erreicht werden kann. Wenn die Biopsie im Kopf eines Patienten entnommen werden soll, wird ein stereotaktischer Rahmen mit Knochenschrauben fest mit dem Kopf des Patienten verbunden. Dieser Rahmen trägt eine Führung zur Führung einer Hohlnadel, die zum Einstechen entlang der vorher geplanten Trajektorie ausgerichtet wird.

Zur Vorbereitung der Biopsieentnahme werden geeignete Photosensibilisatoren, beispielsweise 5-Aminolävulinsäure (5-ALS) und Indocyaningrün (ICG), mit entsprechenden Vorlaufzeiten verabreicht. Die Vorlaufzeit der Gabe von 5-ALS beträgt dabei einige Stunden, um durch Stoffwechselvorgänge die Umwandlung in das fluoreszenzfähige PpIX zu ermöglichen. Die Gabe von ICG erfolgt kurze Zeit vor dem Eingriff, so dass dieser Farbstoff in den das Eingriffsgebiet umgebenden Blutgefäßen in ausreichender Konzentration vorhanden ist. Ggf. wird Fluoreszein ebenfalls intravenös verabreicht.

Sodann werden die oberflächennahen Gewebeschichten und beispielsweise die Schädeldecke zur Vorbereitung des Einstichs geöffnet. Vor dem Einstich wird der als endoskopisch einführbare Sonde ausgebildete distale Endabschnitt des Lichtleiters in die als Hohlnadel ausgebildete Biopsienadel bis zum distalen Ende der Biopsienadel eingeführt. Die bevorzugt nach Art eines Kontaktendoskops ausgebildete Sonde ersetzt somit den für die Einführung üblicherweise eingesetzten Dorn. Hierdurch ist nicht nur das distale Ende der Biopsienadel verschlossen, sondern es wird während des Einführens eine endoskopische Fluoreszenzuntersuchung des jeweils vor der Spitze der Hohlnadel liegenden Gewebes ermöglicht.

Schließlich wird die Biopsienadel mit der eingeführten Sonde, insbesondere unter Führung durch das Stereotaxiesystem entlang der vorbestimmten Trajektorie, in das Gewebe eingeführt. Hierbei wird die Lichterzeugungseinrichtung aktiviert, so dass sequentiell oder gleichzeitig Fluoreszenzanregungslicht in den zur Anregung der Fluoreszenz geeigneten Anregungswellenlängenbereichen erzeugt und in den Lichtleiter eingekoppelt wird, nämlich mit Wellenlängen von 785 nm zur Anregung der ICG-Fluoreszenz und 405 nm zur Anregung der PpIX- und der Autofluoreszenz. Das am distalen Ende der Sonde anliegende Gewebe wird über den als Bildleiter ausgebildeten Lichtleiter, der ggf. ein Objektiv umfassen kann, mit der Fluoreszenzanregungsstrahlung beleuchtet. Die vom Gewebe emittierte Fluoreszenzstrahlung wird direkt in die distale Endfläche des Bildleiters eingekoppelt oder vom Objektiv auf die distale Endfläche des Bildleiters abgebildet, der das Bild zur Strahlteileroptik weiterleitet, wo das Fluoreszenzbild spektral von der Fluoreszenzanregungsstrahlung getrennt wird. Auf dem Bildsensor der CCD-Kamera wird ein Fluoreszenzbild erzeugt, das vom Arzt beim Einführen der Biopsienadel auf einem Bildschirm beobachtet werden kann. Dabei werden die mindestens drei unterschiedlichen angeregten Fluoreszenzmodi voneinander unterscheidbar dargestellt, indem die unterschiedlichen Fluoreszenzen beispielsweise gleichzeitig als unterschiedliche Farben auf einem Farbbildschirm angezeigt werden, oder die unterschiedlichen Fluoreszenzbilder können sequentiell dargestellt werden. Bei einer farbkodierten Darstellung können insbesondere die ICG-Fluoreszenz blau, die Autofluoreszenz grün und die PpIX-Fluoreszenz rot dargestellt werden; wird zusätzlich die Fluoreszenz von Fluoreszein beobachtet, so werden beispielsweise die Autofluoreszenz blau-grün und die Fluoreszein-Fluoreszenz grün dargestellt. Werden die unterschiedlichen Fluoreszenzbilder sequentiell dargestellt, so kann der Arzt wählen, welches Bild aktuell angezeigt werden soll. Der Vorschub der Biopsienadel erfolgt somit unter endoskopischer Sicht, wobei unterschiedliche Fluoreszenzmodi zur Unterscheidung unterschiedlicher Zellarten bzw. unterschiedlicher Gewebearten bzw. Organe visualisiert werden können.

Die ICG-Fluoreszenz gestattet eine Erkennung von am distalen Sondenende anliegenden oder in einer Nachbarschaft des distalen Sondenendes (beispielsweise bis zu einer Entfernung von ca. 1,5 mm) befindlichen Blutgefäßen; eine zusätzliche Gabe von Fluoreszein ermöglicht eine verbesserte Detektion von Blutgefäßen, insbesondere auch des Kapillarbetts und der Mikrozirkulation. Hierdurch ist es möglich, eine Verletzung von Blutgefäßen sicher zu vermeiden, indem die Hohlnadel ggf. zurückgezogen und ein anderer Weg gewählt wird. Insbesondere kann eine Verletzung von Blutgefäßen auch dann vermieden werden, wenn diese Blutgefäße in den zuvor erhobenen 3D-Daten nicht ausreichend erfasst waren oder zwischen der Erfassung und dem Einstich eine Verschiebung des Gewebes stattgefunden hat.

Ferner ist aufgrund der Autofluoreszenz des Gewebes gleichzeitig eine Erkennung des normalen Gewebes und eine Unterscheidung etwa von nekrotischem Tumorgewebe möglich, ebenso wie die Erkennung von vitalem Tumorgewebe durch die PpIX-Fluoreszenz. Bei der Annäherung an den vorgesehenen Ort der Biopsieentnahme kann der Arzt somit jederzeit erkennen, ob sich die Spitze der Biopsienadel in normalem Gewebe, in vitalem Tumorgewebe, beispielsweise im Randbereich des Tumors, oder in nekrotischem Tumorgewebe befindet. Hierdurch kann sichergestellt werden, dass die Biopsie in dem für ein aussagefähiges Biopsieergebnis geeigneten Gewebebereich entnommen wird.

Zur Entnahme der Biopsie wird die Sonde aus der Biopsienadel herausgezogen, eine Biopsiezange in die Biopsienadel bis zu ihrem distalen Ende eingeführt und eine Gewebeprobe entnommen. Dieser Vorgang kann mehrmals wiederholt werden. Schließlich wird die Biopsienadel aus dem Gewebe herausgezogen, und die zuvor geöffneten Gewebeschichten werden wieder verschlossen.

### Bezugszeichenliste

- 1: Lichtleiter
- 2: Faserbündel
- 3: Sonde
- 4: Proximaler Endabschnitt
- 5: Proximale Endfläche
- 6: Distale Endfläche
- 7: Lichtkegel
- 10: Strahlteileroptik
- 11: Erste Kollimatoroptik
- 12: Zweite Kollimatoroptik
- 13: Erster Strahlteiler
- 14: Zweiter Strahlteiler
- 15: Koppeloptik
- 16,: 16` Blockfilter
- 17: Anregungslichtbündel
- 18: Anregungslichtbündel
- 19: Anregungslichtbündel
- 20: Beleuchtungslichtleiter
- 21: Beleuchtungslichtleiter
- 22: Strahlteiler
- 23: Koppeloptik
- 24: Lichtleiter
- 25, 25': Bandpassfilter
- 30: Detektoreinrichtung
- 31: CCD-Kamera
- 32: Kupplung
- 40, 40': Transmissionsfunktion
- 41: Wellenlängenbereich
- 42: Wellenlängenbereich
- 50: Empfindlichkeitsfunktion
- 51: Empfindlichkeitsfunktion
- 52: Empfindlichkeitsfunktion

## Patentansprüche

1. Vorrichtung zur endoskopischen Fluoreszenzdetektion, umfassend eine Lichterzeugungseinrichtung zur Erzeugung einer Fluoreszenzanregungsstrahlung, einen endoskopisch einsetzbaren Lichtleiter (1) zur Weiterleitung der Fluoreszenzanregungsstrahlung von einer proximalen Endfläche (5) des Lichtleiters (1) zu einem zu untersuchenden menschlichen oder tierischen Gewebe und zur Aufnahme und Weiterleitung einer von dem Gewebe emittierten Fluoreszenzstrahlung zur proximalen Endfläche (5) des Lichtleiters (1), eine Detektoreinrichtung (30) zur Detektion der Fluoreszenzstrahlung und eine am proximalen Ende des Lichtleiters (1) angeordnete Strahlteileroptik (10) zur Einkopplung der Fluoreszenzanregungsstrahlung in den Lichtleiter (1) und zur Weiterleitung der Fluoreszenzstrahlung von der proximalen Endfläche (5) des Lichtleiters (1) zur Detektoreinrichtung (30), wobei die Lichterzeugungseinrichtung zur Erzeugung einer solchen Fluoreszenzanregungsstrahlung ausgebildet ist, die zur Anregung der Emission von Fluoreszenzstrahlung des Gewebes in mindestens drei unterschiedlichen Fluoreszenzwellenlängenbereichen geeignet ist und wobei die Detektoreinrichtung (30) zur Unterscheidung der mindestens drei unterschiedlichen Wellenlängenbereiche der Fluoreszenzstrahlung ausgebildet ist.

2. Vorrichtung nach Anspruch 1, **dadurch gekennzeichnet, dass** die Fluoreszenzanregungsstrahlung mindestens einen ersten und einen zweiten Anregungswellenlängenbereich umfasst, wobei der erste Anregungswellenlängenbereich zur Anregung von Fluoreszenzstrahlung in einem ersten Fluoreszenzwellenlängenbereich geeignet ist und der zweite Anregungswellenlängenbereich zur Anregung von Fluoreszenzstrahlung in einem zweiten und einem dritten Fluoreszenzwellenlängenbereich geeignet ist.

3. Vorrichtung nach dem vorhergehenden Anspruch, **dadurch gekennzeichnet, dass** die Fluoreszenzstrahlung im ersten und zweiten Fluoreszenzwellenlängenbereich durch durch mindestens einen Photosensibilisator induzierte Fluoreszenz erzeugt wird und die Fluoreszenzstrahlung im dritten Fluoreszenzwellenlängenbereich durch eine Autofluoreszenz des Gewebes erzeugt wird.

4. Vorrichtung nach dem vorhergehenden Anspruch, **dadurch gekennzeichnet, dass** die Fluoreszenzanregungsstrahlung einen weiteren Anregungswellenlängenbereich umfasst, der zur Anregung von Fluoreszenzstrahlung in einem weiteren Fluoreszenzwellenlängenbereich geeignet ist, wobei die Fluoreszenzstrahlung im weiteren Fluoreszenzwellenlängenbereich durch eine durch einen weiteren Photosensibilisator induzierte Fluoreszenz erzeugt wird.

5. Vorrichtung nach einem der vorhergehenden Ansprüche, **dadurch gekennzeichnet, dass** die Lichterzeugungseinrichtung eine erste und eine zweite Lichtquelle umfasst, wobei die erste Lichtquelle zur Erzeugung einer Fluoreszenzanregungsstrahlung in einem ersten Anregungswellenlängenbereich und die zweite Lichtquelle zur Erzeugung einer Anregungsstrahlung in einem zweiten Anregungswellenlängenbereich ausgebildet ist, und wobei die erste und die zweite Lichtquelle unabhängig voneinander steuerbar sind.

6. Vorrichtung nach einem der vorhergehenden Ansprüche, **dadurch gekennzeichnet, dass** die Strahlteileroptik (10) einen wellenlängenselektiven Strahlteiler umfasst.

7. Vorrichtung nach einem der vorhergehenden Ansprüche, **dadurch gekennzeichnet, dass** die Strahlteileroptik (10) einen im Lichtweg zur Detektoreinrichtung anordenbaren Blockfilter (16, 16') aufweist.

8. Vorrichtung nach einem der vorhergehenden Ansprüche, **dadurch gekennzeichnet, dass** zumindest ein distaler Endabschnitt (3) des Lichtleiters (2) als endoskopisch einführbare Sonde ausgebildet ist oder in einer solchen Sonde aufgenommen ist.

9. Vorrichtung nach dem vorhergehenden Anspruch, **dadurch gekennzeichnet, dass** die Sonde zum Einsetzen in eine Biopsienadel ausgebildet ist.

10. Vorrichtung nach einem der vorhergehenden Ansprüche, **dadurch gekennzeichnet, dass** der Lichtleiter (1) als Bildleiter ausgebildet ist und die Detektoreinrichtung (30) mindestens einen Bildsensor zur Aufnahme eines Fluoreszenzbilds in mindestens einem der mindestens drei unterschiedlichen Fluoreszenzwellenlängenbereiche umfasst.

11. Vorrichtung nach dem vorhergehenden Anspruch, **dadurch gekennzeichnet, dass** am distalen Ende des Lichtleiters (1) ein Objektiv zum Erzeugen eines Bild des Gewebes auf einer distalen Endfläche des Bildleiters vorgesehen ist.

12. Vorrichtung nach Anspruch 10 oder 11, **dadurch gekennzeichnet, dass** die Vorrichtung Anzeigemittel zur Anzeige mindestens eines Fluoreszenzbildes umfasst.

13. Vorrichtung nach einem der Ansprüche 10 bis 12, **dadurch gekennzeichnet, dass** die Lichterzeugungseinrichtung eine Mehrzahl von Lichtquellen zur Erzeugung von Fluoreszenzanregungsstrahlung in mindestens zwei unterschiedlichen Anregungswellenlängenbereichen umfasst und dass die Lichtquellen sequentiell ansteuerbar und mit einer Aufnahmefrequenz des Bildsensors synchronisierbar sind.

14. Verfahren zur endoskopischen Fluoreszenzdetektion, wobei eine Fluoreszenzanregungsstrahlung erzeugt, durch eine am proximalen Ende eines endoskopisch einsetzbaren Lichtleiters (1) angeordnete Strahlteileroptik (10) in eine proximale Endfläche (5) des Lichtleiters (1) eingekoppelt und über den Lichtleiter (1) zu einem zu untersuchenden menschlichen oder tierischen Gewebe weitergeleitet wird, wobei eine von dem Gewebe emittierte Fluoreszenzstrahlung an einem distalen Ende des Lichtleiters (1) aufgenommen und zur proximalen Endfläche (5) des Lichtleiters (1) weitergeleitet und durch die Strahlteileroptik (10) zu einer Detektoreinrichtung (30) weitergeleitet und von dieser detektiert wird, wobei die Fluoreszenzanregungsstrahlung eine Emission von Fluoreszenzstrahlung des Gewebes in mindestens drei unterschiedlichen Fluoreszenzwellenlängenbereichen anregt und die Fluoreszenzstrahlung in jedem der mindestens drei unterschiedlichen Wellenlängenbereiche detektiert wird.

## Claims

1. Apparatus for endoscopic fluorescence detection, comprising a light generation device for generating fluorescence excitation radiation, an optical waveguide (1) which is usable in an endoscopic context for forwarding the fluorescence excitation radiation from a proximal end surface (5) of the optical waveguide (1) to human or animal tissue to be examined and for receiving and forwarding fluorescence radiation emitted by the tissue to the proximal end surface (5) of the optical waveguide (1), a detector device (30) for detecting the fluorescence radiation and a beam splitter optical unit (10) arranged at the proximal end of the optical waveguide (1) for coupling the fluorescence excitation radiation into the optical waveguide (1) and for forwarding the fluorescence radiation from the proximal end surface (5) of the optical waveguide (1) to the detector device (30), wherein the light generation device is embodied to generate such fluorescence excitation radiation which is suitable for exciting the emission of fluorescence radiation of the tissue in at least three different fluorescence wavelength ranges and wherein the detector device (30) is embodied to distinguish the at least three different wavelength ranges of the fluorescence radiation.

2. Apparatus according to Claim 1, **characterized in that** the fluorescence excitation radiation comprises at least a first excitation wavelength range and a second excitation wavelength range, wherein the first excitation wavelength range is suitable for exciting fluorescence radiation in a first fluorescence wavelength range and the second excitation wavelength range is suitable for exciting fluorescence radiation in a second fluorescence wavelength range and in a third fluorescence wavelength range.

3. Apparatus according to the preceding claim, **characterized in that** the fluorescence radiation in the first fluorescence wavelength range and in the second fluorescence wavelength range is generated by fluorescence induced by at least one photosensitizer and the fluorescence radiation in the third fluorescence wavelength range is generated by autofluorescence of the tissue.

4. Apparatus according to the preceding claim, **characterized in that** the fluorescence excitation radiation comprises a further excitation wavelength range which is suitable for exciting fluorescence radiation in a further fluorescence wavelength range, wherein the fluorescence radiation in the further fluorescence wavelength range is generated by fluorescence induced by a further photosensitizer.

5. Apparatus according to one of the preceding claims, **characterized in that** the light generation device comprises a first light source and a second light source, wherein the first light source is embodied to generate fluorescence excitation radiation in a first excitation wavelength range and the second light source is embodied to generate excitation radiation in a second excitation wavelength range, and wherein the first light source and the second light source are controllable independently of one another.

6. Apparatus according to one of the preceding claims, **characterized in that** the beam splitter optical unit (10) comprises a wavelength-selective beam splitter.

7. Apparatus according to one of the preceding claims, **characterized in that** the beam splitter optical unit (10) comprises a block filter (16, 16') which is arrangeable in the light path to the detector device.

8. Apparatus according to one of the preceding claims, **characterized in that** at least a distal end portion (3) of the optical waveguide (2) is embodied as a probe which is insertable in an endoscopic context or is incorporated within such a probe.

9. Apparatus according to the preceding claim, **characterized in that** the probe is embodied for insertion into a biopsy needle.

10. Apparatus according to one of the preceding claims, **characterized in that** the optical waveguide (1) is embodied as an image conductor and the detector device (30) comprises at least one image sensor for recording a fluorescence image in at least one of the at least three different fluorescence wavelength ranges.

11. Apparatus according to the preceding claim, **characterized in that** an objective for generating an image of the tissue at a distal end surface of the image conductor is provided at the distal end of the optical waveguide (1).

12. Apparatus according to Claim 10 or 11, **characterized in that** the apparatus comprises display means for displaying at least one fluorescence image.

13. Apparatus according to one of Claims 10 to 12, **characterized in that** the light generation device comprises a plurality of light sources for generating fluorescence excitation radiation in at least two different excitation wavelength ranges and **in that** the light sources are actuatable in sequence and synchronizable with a recording frequency of the image sensor.

14. Method for endoscopic fluorescence detection, wherein fluorescence excitation radiation is generated, coupled into a proximal end surface (5) of an optical waveguide (1) by way of a beam splitter optical unit (10) arranged at the proximal end of the optical waveguide (1) which is usable in an endoscopic context and forwarded via the optical waveguide (1) to human or animal tissue to be examined, wherein fluorescence radiation emitted by the tissue is received at a distal end of the optical waveguide (1) and forwarded to the proximal end surface (5) of the optical waveguide (1) and forwarded by the beam splitter optical unit (10) to a detector device (30) and detected by the latter, wherein the fluorescence excitation radiation excites an emission of fluorescence radiation of the tissue in at least three different fluorescence wavelength ranges and the fluorescence radiation is detected in each one of the at least three different wavelength ranges.

## Revendications

1. Dispositif de détection de fluorescence endoscopique, comprenant un dispositif générateur de lumière destiné à générer un rayonnement d'excitation de la fluorescence, un conducteur optique (1) pouvant être inséré de manière endoscopique pour acheminer le rayonnement d'excitation de la fluorescence depuis une surface d'extrémité proximale (5) du conducteur optique (1) vers un tissu humain ou animal à examiner et pour acquérir et réacheminer un rayonnement de fluorescence émis par le tissu vers la surface d'extrémité proximale (5) du conducteur optique (1), un dispositif détecteur (30) destiné à détecter le rayonnement de fluorescence et une optique de division de faisceau (10) disposée à l'extrémité proximale du conducteur optique (1), destinée à injecter le rayonnement d'excitation de la fluorescence dans le conducteur optique (1) et à réacheminer le rayonnement de fluorescence de la surface d'extrémité proximale (5) du conducteur optique (1) vers le dispositif détecteur (30), dans lequel le dispositif générateur de lumière est conçu pour générer un rayonnement d'excitation de la fluorescence qui est approprié pour l'excitation de l'émission du rayonnement de fluorescence du tissu dans au moins trois plages de longueurs d'onde de fluorescence différentes et dans lequel le dispositif détecteur (30) est conçu pour différencier les au moins trois plages de longueurs d'onde différentes du rayonnement de fluorescence.

2. Dispositif selon la revendication 1, **caractérisé en ce que** le rayonnement d'excitation de la fluorescence comprend au moins une première et une deuxième plage de longueurs d'onde d'excitation, dans lequel la première plage de longueurs d'onde d'excitation est appropriée pour l'excitation d'un rayonnement de fluorescence dans une première plage de longueurs d'onde de fluorescence et la deuxième plage de longueurs d'onde d'excitation est appropriée pour l'excitation d'un rayonnement de fluorescence dans des deuxième et une troisième plages de longueurs d'onde de fluorescence.

3. Dispositif selon la revendication précédente, **caractérisé en ce que** le rayonnement de fluorescence dans les première et deuxième plages de longueurs d'onde de fluorescence est généré par une fluorescence induite par au moins un photosensibilisateur et **en ce que** le rayonnement de fluorescence dans la troisième plage de longueurs d'onde de fluorescence est généré par une auto-fluorescence du tissu.

4. Dispositif selon la revendication précédente, **caractérisé en ce que** le rayonnement d'excitation de la fluorescence comprend une autre plage de longueurs d'onde d'excitation qui est appropriée pour l'excitation d'un rayonnement de fluorescence dans une autre plage de longueurs d'onde de fluorescence, dans lequel le rayonnement de fluorescence est généré dans l'autre plage de longueurs d'onde de fluorescence par une fluorescence induite par un autre photosensibilisateur.

5. Dispositif selon l'une quelconque des revendications précédentes, **caractérisé en ce que** le dispositif générateur de lumière comprend des première et deuxième sources de lumière, dans lequel la première source de lumière est conçue pour générer un rayonnement d'excitation de la fluorescence dans une première plage de longueurs d'onde d'excitation et la deuxième source de lumière est conçue pour générer un rayonnement d'excitation dans une deuxième plage de longueurs d'onde d'excitation et dans lequel les première et deuxième sources de lumière peuvent être commandées indépendamment l'une de l'autre.

6. Dispositif selon l'une quelconque des revendications précédentes, **caractérisé en ce que** l'optique de division de faisceau (10) comprend un diviseur de faisceau sélectif en longueur d'onde.

7. Dispositif selon l'une quelconque des revendications précédentes, **caractérisé en ce que** l'optique de division de faisceau (10) comprend un filtre-bloc (16, 16') pouvant être disposé sur le chemin optique vers le dispositif détecteur.

8. Dispositif selon l'une quelconque des revendications précédentes, **caractérisé en ce qu'**au moins une section d'extrémité distale (3) du conducteur optique (2) est réalisée sous la forme d'une sonde pouvant être insérée de manière endoscopique ou est reçue dans une sonde de ce type.

9. Dispositif selon la revendication précédente, **caractérisé en ce que** la sonde est réalisée sous la forme d'un insert introduit dans une aiguille de biopsie.

10. Dispositif selon l'une quelconque des revendications précédentes, **caractérisé en ce que** le conducteur optique (1) est réalisé sous la forme d'un conducteur d'image et **en ce que** le dispositif détecteur (30) comprend au moins un capteur d'image destiné à acquérir une image de fluorescence dans au moins l'une des au moins trois plages de longueurs d'onde de fluorescence différentes.

11. Dispositif selon la revendication précédente, **caractérisé en ce qu'**un objectif destiné à générer une image du tissu sur une surface d'extrémité distale du conducteur d'image est prévu à l'extrémité distale du conducteur optique (1).

12. Dispositif selon la revendication 10 ou 11, **caractérisé en ce que** le dispositif comprend des moyens d'affichage destinés à afficher au moins une image de fluorescence.

13. Dispositif selon l'une quelconque des revendications 10 à 12, **caractérisé en ce que** le dispositif générateur de lumière comprend une pluralité de sources de lumière destinées à générer un rayonnement d'excitation de la fluorescence dans au moins trois plages de longueurs d'onde d'excitation différentes et **en ce que** les sources de lumière peuvent être attaquées de manière séquentielle et peuvent être synchronisées avec une fréquence d'acquisition du capteur d'image.

14. Procédé de détection de fluorescence endoscopique, dans lequel un rayonnement d'excitation de la fluorescence est généré, en ce qu'il est injecté à travers une optique de division de faisceau (10) disposé à une extrémité proximale d'un conducteur optique (1) pouvant être inséré de manière endoscopique dans une surface d'extrémité proximale (5) du conducteur optique (1) et en ce qu'il est réacheminé par l'intermédiaire du conducteur optique (1) vers un tissu humain ou animal à examiner, dans lequel un rayonnement de fluorescence émis par le tissu est acquis à une extrémité distale du conducteur optique (1) et est réacheminé vers la surface d'extrémité proximale (5) du conducteur optique (1) et est réacheminé par l'intermédiaire de l'optique de division de faisceau (10) vers un dispositif détecteur (30) et est détecté par celui-ci, dans lequel le rayonnement d'excitation de la fluorescence excite une émission d'un rayonnement de fluorescence du tissu dans au moins trois plages de longueurs d'onde de fluorescence différentes et le rayonnement de fluorescence est détecté dans chacune des au moins trois plages de longueurs d'onde différentes.
